# EUROPEAN PATENT SPECIFICATION

(11) **EP 4 439 477 B1**
(45) Date of publication and mention of the grant of the patent: **29.04.2026**
(21) Application number: 23164433.7
(22) Date of filing: 27.03.2023
(51) Int. Cl.: G06V 10/143, G06V 10/46, G06V 10/75, G06V 10/80, G06V 20/52, G06V 20/59, G06V 40/10, G06V 40/20, G06T 7/73, G05D 1/00, A61B 5/18, B64D 45/00

(54) **PILOT POSTURE RECOGNITION SYSTEM AND METHOD**
PILOTENHALTUNGSERKENNUNGSSYSTEM UND -VERFAHREN
SYSTÈME ET PROCÉDÉ DE RECONNAISSANCE DE POSTURE DE PILOTE

(43) Date of publication of application: 02.10.2024
(73) Proprietor: Rockwell Collins, Inc., Cedar Rapids, IA 52498 (US)
(72) Inventor: YARLAPATI GANESH, Naresh, Cork, T12 X295 (IE); RIVERSO, Stefano, Cork City, T23 F409 (IE)
(74) Representative: Dehns

(56) References cited:
- US-A1- 2019 090 800
- US-A1- 2022 230 522
- US-A1- 2022 265 187

## Description

### TECHNICAL FIELD

The subject-matter disclosed herein relates to a pilot posture recognition system, e.g. in a cockpit of an aircraft. Particularly, the subject-matter disclosed herein relates to systems and methods for the detection/recognition of pilot posture, pilot position, pilot fall and pilot-object interaction.

### BACKGROUND

Pilot state assessment systems may be used to assess the state of a pilot and/or co-pilot during operation of an aircraft. The position and posture of a pilot and/or co-pilot is an important factor for identifying/predicting fatigue and incapacitation situations during a flight - for example, slouching and/or a loose head position (neck bending) can be indicators that a pilot is tired or asleep. Thus, the inventors have identified a need for an improved pilot monitoring system to better detect and recognise the state/activity of a pilot and/or a co-pilot.

US 2019/090800 A1 discloses a pilot health monitoring system configured to collect information regarding the pilot's physiological and/or physical characteristics and information regarding the state of the aircraft. This information is analysed and used to determine the health condition of the pilot and/or a state of the aircraft. The system is further configured to provide warnings and/or commands as a function of this information.

US 2022/230522 A1 discloses a pilot monitoring system including a camera configured to capture a plurality of digital images of an aircraft cockpit and a processor configured to process the captured images. The processed images are used to determine an alertness state of the pilot based on detected facial features of the pilot.

US 2022/265187 A1 discloses a system for monitoring the operation status of an aircraft crew. The system determines at least one pilot state based on first monitoring data and second monitoring data at difference assurance levels.

### SUMMARY

In a first aspect, there is provided a system comprising: one or more imaging devices configured to collect image data of a cockpit area of an aircraft; a pilot detection module configured to determine a presence of one or more pilots in the cockpit area based on the image data; and a pilot posture module configured to determine three-dimensional posture of the one or more pilots based on the image data, wherein the pilot posture module is capable of determining that a pilot is falling or has fallen. The system comprises an object detection module configured to determine a presence and three-dimensional position of one or more objects in the cockpit area based on the image data, and a pilot-object module configured to, if the pilot posture module detects that one or more pilots has fallen, determine that a pilot-object interaction event has occurred as a result of the fall based on the three-dimensional posture of the one or more pilots and the three-dimensional positions of the one or more objects.

As used herein, the term 'module' should be understood to mean a 'software module', e.g. a software component comprising code corresponding to a part of or a whole of a program. A module is configured to be executed by/run on one or more processors of the system.

The one or more imaging devices may include at least one conventional CCD or CMOS based digital camera having a two-dimensional array of photosensitive pixels, optionally provided with the capability to determine range or depth (such as through one or more phase detect elements). The photosensitive pixels are capable of sensing electromagnetic radiation in the visible wavelength range. Alternatively, the one or more imaging devices may include at least one three-dimensional camera such as a time-of-flight camera (e.g. a camera comprising, or associated with, a time-of-flight sensor including, for example, an IR emitter and receiver) or other scanning or range-imaging camera capable of imaging a scene in three dimensions. Alternatively, the one or more imaging devices may include a pair of like two-dimensional cameras operating in a stereo configuration and calibrated to extract depth. The one or more imaging devices comprise a two-dimensional digital camera (configured to image in the visible wavelength range) and a time-of-flight sensor (depth sensor), optionally including an IR emitter and receiver.

The one or more imaging devices may comprise a plurality of two-dimensional digital cameras (configured to image in the visible wavelength range) and a plurality of time-of-flight sensors (optionally each including an IR emitter and receiver).

The modules and models of the system described herein may be configured (e.g. trained) to process/handle image data from a number of different imaging device configurations. In other words, the modules and models may be presented (during training) with a plurality of different view configurations of the cockpit, each view configuration comprising one or more image views (with each image view being provided by a particular imaging device in a particular position). In this way, the modules of the system may be configured to be 'view independent', in that they can process/handle image data from any imaging device configuration (i.e. one or more imaging devices arranged in any position(s)) and yet still provide robust and accurate evaluations of the scene in the cockpit.

The system may include a data buffer (or frame buffer) to temporarily store the image data. The image data may be provided from the data buffer to one or more modules of the system, the one or more modules being configured to process the data and derive conclusions. As described above, the system may comprise one or more processors on which the one or more modules may be run.

The pilot detection module may comprise a pre-trained pilot detection machine learning model. In other words, the pilot detection machine learning model may be an object detection model configured (e.g. trained) to detect/identify a person (e.g. a pilot) in the cockpit.

As noted above, the system comprises an object detection image module configured to determine a presence and three-dimensional position of one or more objects in the cockpit area based on the image data. In the present context, an 'object' is understood to refer to inanimate cockpit objects, and in particular to flight deck objects. Relevant objects may include, for example, control levers, control panels including buttons and switches, sidesticks, control pedals, and control wheels/yokes/tillers.

The object detection module may comprise a pre-trained flight deck object detection machine learning model. In other words, the flight deck object machine learning model may be configured (e.g. trained) to detect/identify an object (e.g. a control stick of a flight deck) in the cockpit.

As noted above, the system comprises a pilot-object module configured to, if the pilot posture module detects that one or more pilots have fallen, determine that a pilot-object interaction event has occurred as a result of the fall based on the three-dimensional posture of the one or more pilots and the three-dimensional positions of the one or more objects.

Detection of a pilot-object interaction may be carried out by a pre-trained LSTM network model. Herein, an LSTM network model is a deep neural network model capable of remembering a plurality of past image frames, and predicting a plurality of future image frames. The pilot-object LSTM network model may have been pre-trained using a dataset comprising videos of pilot interactions with flight deck objects.

The system may be configured to pass the determined three-dimensional posture and/or the determined pilot-object interaction event to an alert system.

The alert system may be configured to: verify that an action has been input into a flight system log of the aircraft as a result of the determined pilot-object interaction event; and alert a control system of the aircraft that the action input was a result of the determined pilot-object interaction event.

The alert system may be configured to alert aircraft crew and/or ground control and/or Air Traffic Control that one or more pilots have fallen.

The pilot posture module may comprise a depth information module configured to extract depth information from the image data; and a pilot pose module configured to identify a plurality of two-dimensional keypoints corresponding to features of the one or more pilots in the image data, wherein the pilot posture module is configured to combine the depth information and the two-dimensional keypoints to determine a three-dimensional posture of the one or more pilots in the cockpit.

The pilot pose module may identify the plurality of two-dimensional keypoints by use of a pre-trained human pose estimation model.

The two-dimensional keypoints may comprise the location of the pilot's eyes, ears, nose, shoulders, elbows, wrists, and hips.

Determination of the three-dimensional posture of the one or more cockpits may be carried out by a pre-trained LSTM network model. The pilot posture LSTM network model may have been pre-trained using a dataset comprising images and videos e.g. of one or more pilots in the cockpit.

The LSTM network model may be configured (e.g. trained) to determine feature relationships between key points (i.e. the three-dimensional distance and angles between them) and to utilise the determined feature relationships to identify a three-dimensional posture. During (pre-)training, the LSTM network model will learn to recognize or classify different postures based on characteristic/distinctive feature relationships.

In another aspect, there is provided a method, comprising: collecting, using one or more imaging devices, image data of a cockpit area of an aircraft; determining, using a pilot detection module, a presence of one or more pilots in the cockpit area based on the image data; determining, using a pilot posture image module, three-dimensional posture of the one or more pilots based on the image data; determining, using the pilot posture module, that a pilot is falling or has fallen; determining, using an object detection image module, a presence and three-dimensional position of one or more objects in the cockpit area based on the image data; determining, using a pilot-object module, that a pilot-object interaction event has occurred as a result of the fall based on the three-dimensional posture of the one or more pilots and the three-dimensional positions of the one or more objects.

The method may comprise use of the system of the first aspect, optionally including any of the optional features thereof.

As noted above, after determining that one or more pilots have fallen, the method comprises determining, using a pilot-object module, that a pilot-object interaction event occurred as a result of the fall based on the three-dimensional posture of the one or more pilots and the three-dimensional positions of the one or more objects before and after, and/or during the fall. In other words, if the pilot posture recognition module identifies a fall event of one or more pilots, the pilot-object module will determine (based on the three-dimensional posture of the one or more pilots and the three-dimensional positions of the one or more objects at the time of the fall event) whether a pilot-object interaction event occurred (e.g. identifying whether the pilot(s) knocked any controls as they fell down).

The method may comprise verifying, using an alert system, that an action has been input into a flight system log of the aircraft as a result of the determined pilot-object interaction event; and alerting, using the alert system, a control system of the aircraft that the action input was a result of the determined pilot-object interaction event.

The method may comprise alerting, using an alert system, aircraft crew and/or ground control and/or Air Traffic Control that one or more pilots have fallen.

The method may comprise extracting, using a depth information module of the pilot posture image module, depth information from the image data; identifying, using a pilot pose module of the pilot posture image module, a plurality of two-dimensional keypoints corresponding to features of the one or more pilots in the image data; and combining, using the pilot posture image module, the depth information and the two-dimensional keypoints to determine three-dimensional posture of the one or more pilots in the cockpit.

### BRIEF DESCRIPTION OF THE DRAWINGS

Fig. 1 shows an example of a pilot state monitoring system.
Fig. 2 shows an example of a pilot posture recognition system.
Fig. 3 shows an example of a pilot fall and object interaction detection system.

### DETAILED DESCRIPTION

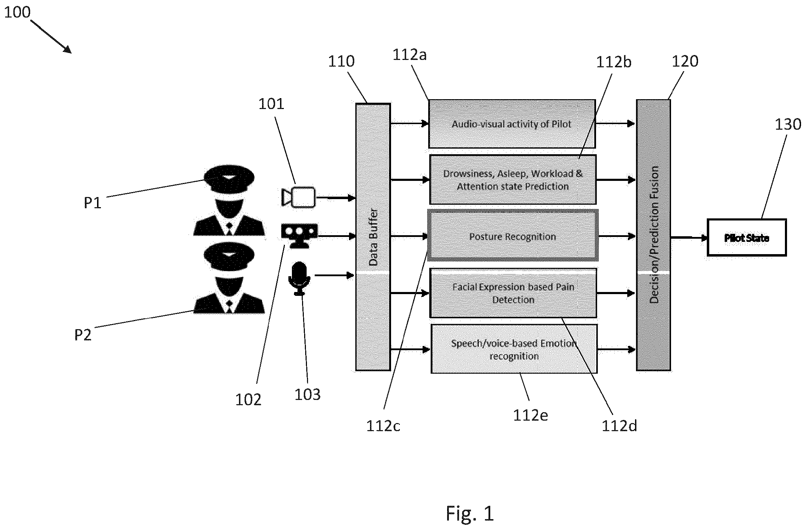

Figure 1 generally shows an example of a pilot state monitoring system 100. The a pilot state monitoring system 100 is configured to monitor the activity of one or more pilots (e.g. P1 and P2) during flight. The pilot state monitoring system 100 comprises one or more imaging devices including, for example, a visible light camera 101 and an infrared emitter/receiver 102 which acts as a time of flight detector. Optionally, the pilot state monitoring system 100 may include one or more audio collectors 103 (e.g. a microphone). The infrared/depth camera 102 may assist in extracting and reconstructing a three-dimensional scene of the cockpit where the one or more pilots P1/P2 are situated.

As shown in Figure 1, data from at least one of the image collector 101, the infrared/depth device 102 and the audio collector 103 may be forwarded to be processed through a data buffer 110 to temporarily store data before it is processed. The data buffer 110 can buffer the data from at least one of the image collector 101, the infrared emitter/receiver 102, and the audio collector 103. The data may then be extrapolated from the data buffer 110 in order to determine the pilot state 130. For example, data may be passed to one of the following subsystems: a) audio-visual pilot activity recognition subsystem 112a, b) pilot drowsiness recognition subsystem 112b, c) pilot posture recognition subsystem 112c, d) pilot pain recognition subsystem 112d, and e) pilot emotion recognition subsystem 112e. After one or more of these subsystems 112a-112e have processed the received data, the processed data is sent through to a decision module 120 where the processed data is analysed to determine a pilot state 130.

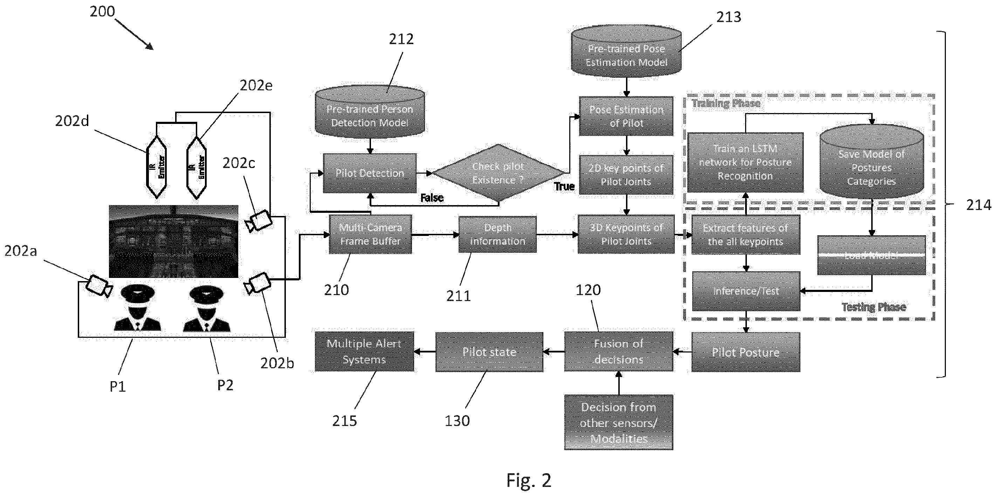

Figure 2 shows further detail of a pilot posture recognition subsystem 200. As shown in Figure 2, a cockpit area may include one or more pilots (e.g. P1 and P2). As shown in Figure 2, the cockpit area includes one or more imaging devices 202 including three RGB cameras (e.g. 202a, 202b, 202c) and two IR/depth sensors (e.g. 202d and 202e). In this example, the one or more imaging devices 202 are located to face the front side of the one or more pilots P1 and/or P2. The one or more imaging devices collect image data from the cockpit area, e.g. of the one or more pilots P1, P2. The image data may be taken during flight.

The modules and models of the systems 100, 200 described herein may be configured (e.g. trained) to process/handle image data from a number of different imaging device configurations. In other words, the modules and models may be presented (during training) with a plurality of different view configurations of the pilot(s)/cockpit, each view configuration comprising one or more image views (with each image view being provided by a particular imaging device 202 in a particular position/orientation). In this way, the modules of the system 100, 200 may be configured to be 'view independent', in that they can process/handle image data from any imaging device configuration (i.e. one or more imaging devices 202 arranged in any position(s)/orientation(s)) and yet still provide robust and accurate evaluations of the scene in the cockpit.

As can be seen in Figure 2, the image data is provided to a frame buffer 210 where the image data is temporarily stored for processing. The image data provided to the frame buffer 210 is transposed to two different branches. In the first branch, the image data is provided from the frame buffer 210 to a depth information module 211 that is configured to extract depth information from the image data. In the second branch, the image data is provided to a pilot detection module 212 to determine a presence of one or more of the pilots P1, P2. If the presence of one or more of the pilots P1, P2 is detected, the image data is passed to a pilot pose module 213 to estimate a pose of the one or more pilots P1, P2. The poses are detected by identifying, using a (pre-trained) human pose estimation model, a plurality two-dimensional keypoints corresponding to features of the one or more pilots P1, P2. The keypoint features include the pilot's eyes, nose, ears, shoulders, elbows, wrists, and hips.

The processed image data from the depth information module 211 and the processed image data from the pilot pose module 213 are then combined by the posture position module 214 to create a three-dimensional reconstruction of the pilot's pose (e.g. to determine three-dimensional positions of the keypoints of the one or more pilots in the cockpit). The three-dimensional keypoints of the one or more pilots in the cockpit are then fed into a LSTM network model of the posture position module 214. The LSTM network model is configured, e.g. (pre-trained), to determine feature relationships between the key points (i.e. the three-dimensional distance and angles between each key point) and to utilise the determined feature relationships to identify a three-dimensional posture. During training, the LSTM network model will learn to recognize or classify different postures based on characteristic/distinctive feature relationships. Accordingly, the LSTM network model is configured, e.g. trained, to recognise/infer postures (e.g. postures including at least head/neck leaning forward, backward, to right, or to left, body leaning to right or to left, normal upright, slouching, sliding down seat, falling off seat, etc.) of the one or more pilots from the three-dimensional keypoints.

Once the pilot posture(s) have been determined by the LSTM network of the pilot posture position module 214, the information is sent to the decision module 120 and/or an alert system/module 215 which may be additionally provided with information from other sensors and subsystems in the system (e.g. from subsystems 112a-e). The decision module 120 and/or alert system/module 215 can then determine a final pilot state 130 and decide if an alert needs to be raised.

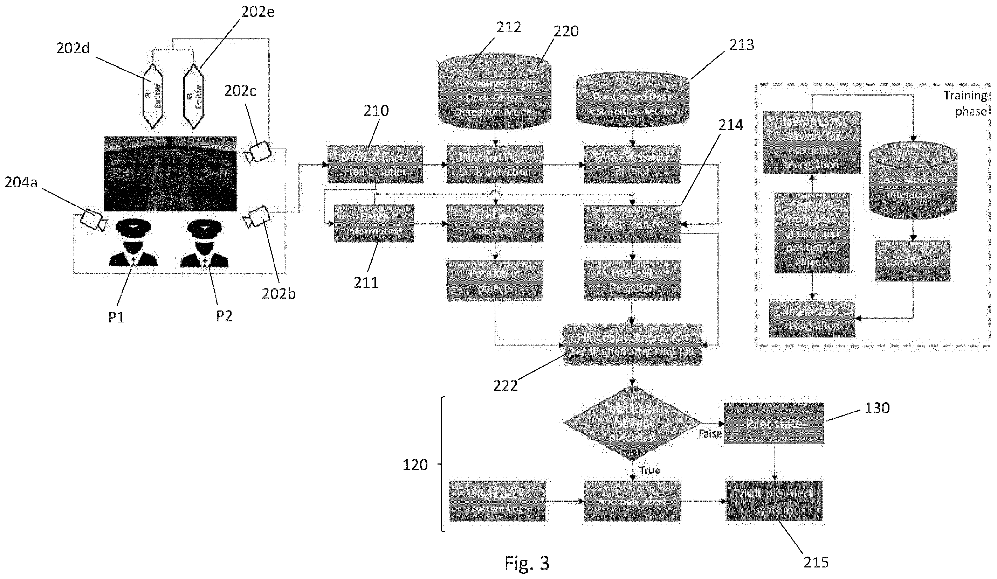

Another exemplary usage of the system is shown in Figure 3. In this example, as before, the image data provided to the frame buffer 210 is transposed to two different branches. However, in this case, in the second branch the pilot detection module 212 further comprises an object detection module 220 configured to determine a presence of one or more objects in the cockpit (e.g. control levers, control panels including buttons and switches, sidesticks/joysticks, control pedals, and control wheels/yokes/tillers). If the presence of one or more objects are detected, the object detection module 220 utilises the processed image data from the depth information module 211 to create a three-dimensional reconstruction of the scene (e.g. to determine three-dimensional positions of the objects in the cockpit).

In the event that the system determines that one or more pilots have fallen (e.g. the posture position indicates that a pilot is falling/has fallen), the processed image data from the object detection module 220 (e.g. the three-dimensional object positions) and the processed image data from the posture position module 214 (e.g. the three-dimensional postures of the one or more pilots P1, P2) are passed to the pilot-object module 222. The pilot-object interaction module 222, as shown in Figure 3, comprises an LSTM network configured, e.g. trained, to recognise when a pilot-object interaction has occurred based on the three-dimensional relative positions of the pilot(s) P1, P2 and the objects. Therefore, for example, the pilot-object module 222 is able to determine if a pilot who has fallen (for any reason) has fallen onto or knocked the flight controls.

If the system determines that a pilot-object interaction has occurred after a fall, then this information is passed to the decision module 120 and/or alert system/module 215. The decision module 120 and/or alert system 215 is configured to check the time at which the determined pilot-object interaction event occurred and identify if an action was input to a flight system log at the time of the determined pilot-object interaction event. If an action was input to the flight system log at the same time as a determined pilot-object interaction event, then the alert system 215 is configured to alert an aircraft management system/control system that the action input was a result of the determined pilot-object interaction. Accordingly, the aircraft management system/control system may be instructed to automatically address the action input (e.g. to rectify or undo the action performed by the inadvertent interaction with the flight controls), or notify aircraft crew (e.g. the remaining one or more pilots) and/or ground control and/or Air Traffic Control. Similarly, the alert system 215 may alert aircraft crew and/or ground control and/or Air Traffic Control that one or more pilots have fallen (e.g. even if a pilot-object interaction has not occurred).

In other words, the system 200 is capable of recognising a first anomalous event (a pilot fall) and instructing the alert system/module 215 to raise a corresponding alert (e.g. to notify at least one of the aircraft management system, aircraft crew, ground control, and Air Traffic Control), and capable of recognising a second anomalous event (a pilot-object interaction after a pilot fall) and instructing the alert system/module 215 to check if a flight action input was a result of the determined pilot-object interaction and, if so, to raise a corresponding alert (e.g. to notify at least one of the aircraft management system, aircraft crew, ground control, and Air Traffic Control).

Although this disclosure has been described in terms of preferred examples, it should be understood that these examples are illustrative only and that the claims are not limited to those examples. Those skilled in the art will be able to make modifications and alternatives in view of the disclosure which are contemplated as falling within the scope of the appended claims.

## Claims

1. A system (200), comprising:
one or more imaging devices (202) configured to collect image data of a cockpit area of an aircraft;
a pilot detection module (212) configured to determine a presence of one or more pilots (P1, P2) in the cockpit area based on the image data;
a pilot posture module (214) configured to determine three-dimensional posture of the one or more pilots based on the image data, wherein the pilot posture module (214) is capable of determining that a pilot is falling or has fallen;
an object detection module (220) configured to determine a presence and three-dimensional position of one or more objects in the cockpit area based on the image data; and
a pilot-object module (222) configured to, if the pilot posture module (214) detects that one or more pilots has fallen, determine that a pilot-object interaction event has occurred as a result of the fall based on the three-dimensional posture of the one or more pilots (P1, P2) and the three-dimensional positions of the one or more objects.

2. The system of claim 1, wherein the system (200) is configured to pass the determined three-dimensional posture and/or the determined pilot-object interaction event to an alert system (120, 215).

3. The system of claim 2, wherein the alert system (120, 215) is configured to:
verify that an action has been input into a flight system log of the aircraft as a result of the determined pilot-object interaction event; and
alert a control system of the aircraft that the action input was a result of the determined pilot-object interaction event.

4. The system of claim 2 or 3, wherein the alert system (120, 215) is configured to:
alert aircraft crew and/or ground control and/or Air Traffic Control that one or more pilots (P1, P2) have fallen.

5. The system of any preceding claim, wherein the pilot posture module (214) comprises:
a depth information module (211) configured to extract depth information from the image data; and
a pilot pose module (213) configured to identify a plurality of two-dimensional keypoints corresponding to features of the one or more pilots (P1, P2) in the image data,
wherein the pilot posture module (214) is configured to combine the depth information and the two-dimensional keypoints to determine three-dimensional posture of the one or more pilots in the cockpit.

6. A method, comprising:
collecting, using one or more imaging devices (202), image data of a cockpit area of an aircraft;
determining, using a pilot detection module (212), a presence of one or more pilots in the cockpit area based on the image data;
determining, using a pilot posture module (214), three-dimensional posture of the one or more pilots (P1, P2) based on the image data;
determining, using the pilot posture module (214), that a pilot is falling or has fallen;
determining, using an object detection image module (220), a presence and three-dimensional position of one or more objects in the cockpit area based on the image data;
determining, using a pilot-object module (222), that a pilot-object interaction event has occurred as a result of the fall based on the three-dimensional posture of the one or more pilots (P1, P2) and the three-dimensional positions of the one or more objects.

7. The method of claim 6, comprising:
verifying, using an alert system (120, 215), that an action has been input into a flight system log of the aircraft as a result of the determined pilot-object interaction event; and
alerting, using the alert system (120, 215), a control system of the aircraft that the action input was a result of the determined pilot-object interaction event.

8. The method of claims 6 or 7, comprising:
alerting, using an alert system (120, 215), aircraft crew and/or ground control and/or Air Traffic Control that one or more pilots have fallen.

9. The method of any of claims 6 to 8, comprising:
extracting, using a depth information module (211) of the pilot posture module (214), depth information from the image data;
identifying, using a pilot pose module (213) of the pilot posture module (214), a plurality of two-dimensional keypoints corresponding to features of the one or more pilots (P1, P2) in the image data; and
combining, using the pilot posture module (214), the depth information and the two-dimensional keypoints to determine three-dimensional posture of the one or more pilots in the cockpit.

## Patentansprüche

1. System (200), umfassend:
eine oder mehrere Bildgebungsvorrichtungen (202), die konfiguriert sind, Bilddaten eines Cockpitbereichs eines Flugzeugs zu sammeln;
ein Pilotendetektionsmodul (212), das konfiguriert ist, basierend auf den Bilddaten eine Anwesenheit eines oder mehrerer Piloten (P1, P2) in dem Cockpitbereich zu bestimmen;
ein Pilotenhaltungsmodul (214), das konfiguriert ist, basierend auf den Bilddaten eine dreidimensionale Haltung des einen oder der mehreren Piloten zu bestimmen, wobei das Pilotenhaltungsmodul (214) in der Lage ist, zu bestimmen, dass ein Pilot fällt oder gefallen ist;
ein Objektdetektionsmodul (220), das konfiguriert ist, basierend auf den Bilddaten ein Vorhandensein und eine dreidimensionale Position eines oder mehrerer Objekte in dem Cockpitbereich zu bestimmen; und
ein Pilot-Objekt-Modul (222), das konfiguriert ist, wenn das Pilotenhaltungsmodul (214) detektiert, dass ein oder mehrere Piloten gefallen sind, basierend auf der dreidimensionalen Haltung des einen oder der mehreren Piloten (P1, P2) und den dreidimensionalen Positionen des einen oder der mehreren Objekte zu bestimmen, dass als Folge des Falls ein Pilot-Objekt-Interaktionsereignis aufgetreten ist.

2. System nach Anspruch 1, wobei das System (200) konfiguriert ist, die bestimmte dreidimensionale Haltung und/oder das bestimmte Pilot-Objekt-Interaktionsereignis an ein Warnsystem (120, 215) weiterzugeben.

3. System nach Anspruch 2, wobei das Warnsystem (120, 215) konfiguriert ist zum:
Verifizieren, dass als Folge des bestimmten Pilot-Objekt-Interaktionsereignisses eine Aktion in ein Flugsystemprotokoll des Flugzeugs eingegeben wurde; und
Warnen eines Steuersystems des Flugzeugs, dass die Aktionseingabe eine Folge des bestimmten Pilot-Objekt-Interaktionsereignisses war.

4. System nach Anspruch 2 oder 3, wobei das Warnsystem (120, 215) konfiguriert ist zum:
Warnen einer Flugzeugbesatzung und/oder Bodenkontrolle und/oder Flugsicherung, dass ein oder mehrere Piloten (P1, P2) gefallen sind.

5. System nach einem der vorhergehenden Ansprüche, wobei das Pilotenhaltungsmodul (214) umfasst:
ein Tiefeninformationsmodul (211), das konfiguriert ist, Tiefeninformationen aus den Bilddaten zu extrahieren; und
ein Pilotenposenmodul (213), das konfiguriert ist, eine Vielzahl von zweidimensionalen Schlüsselpunkten zu identifizieren, die Merkmalen des einen oder der mehreren Piloten (P1, P2) in den Bilddaten entsprechen,
wobei das Pilotenhaltungsmodul (214) konfiguriert ist, die Tiefeninformationen und die zweidimensionalen Schlüsselpunkte zu kombinieren, um eine dreidimensionale Haltung des einen oder der mehreren Piloten in dem Cockpit zu bestimmen.

6. Verfahren, umfassend:
Sammeln, unter Verwendung einer oder mehrerer Bildgebungsvorrichtungen (202), von Bilddaten eines Cockpitbereichs eines Flugzeugs;
Bestimmen, unter Verwendung eines Pilotendetektionsmoduls (212), einer Anwesenheit eines oder mehrerer Piloten in dem Cockpitbereich basierend auf den Bilddaten;
Bestimmen, unter Verwendung eines Pilotenhaltungsmoduls (214), einer dreidimensionalen Haltung des einen oder der mehreren Piloten (P1, P2) basierend auf den Bilddaten;
Bestimmen, unter Verwendung des Pilotenhaltungsmoduls (214), dass ein Pilot fällt oder gefallen ist;
Bestimmen, unter Verwendung eines Objektdetektionsbildmoduls (220), eines Vorhandenseins und einer dreidimensionalen Position eines oder mehrerer Objekte in dem Cockpitbereich basierend auf den Bilddaten;
Bestimmen, unter Verwendung eines Pilot-Objekt-Moduls (222), basierend auf der dreidimensionalen Haltung des einen oder der mehreren Piloten (P1, P2) und den dreidimensionalen Positionen des einen oder der mehreren Objekte, dass als Folge des Falls ein Pilot-Objekt-Interaktionsereignis aufgetreten ist.

7. Verfahren nach Anspruch 6, umfassend:
Verifizieren, unter Verwendung eines Warnsystems (120, 215), dass als Folge des bestimmten Pilot-Objekt-Interaktionsereignisses eine Aktion in ein Flugsystemprotokoll des Flugzeugs eingegeben wurde; und
Warnen, unter Verwendung des Warnsystems (120, 215), eines Steuersystems des Flugzeugs, dass die Aktionseingabe eine Folge des bestimmten Pilot-Objekt-Interaktionsereignisses war.

8. Verfahren nach Anspruch 6 oder 7, umfassend:
Warnen, unter Verwendung eines Warnsystems (120, 215), einer Flugzeugbesatzung und/oder Bodenkontrolle und/oder Flugsicherung, dass ein oder mehrere Piloten gefallen sind.

9. Verfahren nach einem der Ansprüche 6 bis 8, umfassend:
Extrahieren, unter Verwendung eines Tiefeninformationsmoduls (211) des Pilotenhaltungsmoduls (214), von Tiefeninformationen aus den Bilddaten;
Identifizieren, unter Verwendung eines Pilotenposenmoduls (213) des Pilotenhaltungsmoduls (214), einer Vielzahl von zweidimensionalen Schlüsselpunkten, die Merkmalen des einen oder der mehreren Piloten (P1, P2) in den Bilddaten entsprechen; und
Kombinieren, unter Verwendung des Pilotenhaltungsmoduls (214), der Tiefeninformationen und der zweidimensionalen Schlüsselpunkte, um eine dreidimensionale Haltung des einen oder der mehreren Piloten in dem Cockpit zu bestimmen.

## Revendications

1. Système (200) comprenant :
un ou plusieurs dispositifs d'imagerie (202) configurés pour collecter des données d'image d'une zone de cockpit d'un aéronef ;
un module de détection de pilote (212) configuré pour déterminer une présence d'un ou de plusieurs pilotes (P1, P2) dans la zone de cockpit sur la base des données d'image ;
un module de posture de pilote (214) configuré pour déterminer la posture tridimensionnelle des un ou plusieurs pilotes sur la base de données d'image, dans lequel le module de posture de pilote (214) est capable de déterminer qu'un pilote est en train de tomber ou est tombé ;
un module de détection d'objets (220) configuré pour déterminer une présence et une position tridimensionnelle d'un ou de plusieurs objets dans la zone de cockpit à partir des données d'image ; et
un module pilote-objet (222) configuré pour, si le module de posture de pilote (214) détecte qu'un ou plusieurs pilotes sont tombés, déterminer qu'un événement d'interaction pilote-objet s'est produit à la suite de la chute sur la base de la posture tridimensionnelle des un ou plusieurs pilotes (P1, P2) et des positions tridimensionnelles des un ou plusieurs objets.

2. Système selon la revendication 1, dans lequel le système (200) est configuré pour transmettre la posture tridimensionnelle déterminée et/ou l'événement d'interaction pilote-objet déterminé à un système d'alerte (120, 215).

3. Système selon la revendication 2, dans lequel le système d'alerte (120, 215) est configuré pour :
vérifier qu'une action a été enregistrée dans un journal de bord de système de vol de l'aéronef à la suite de l'événement d'interaction pilote-objet déterminé ; et
alerter un système de contrôle de l'aéronef que l'action saisie était un résultat de l'événement d'interaction pilote-objet déterminé.

4. Système selon la revendication 2 ou 3, dans lequel le système d'alerte (120, 215) est configuré pour :
alerter l'équipage d'aéronef et/ou le contrôle au sol et/ou le contrôle aérien qu'un ou plusieurs pilotes (P1, P2) sont tombés.

5. Système selon une quelconque revendication précédente, dans lequel le module de posture de pilote (214) comprend :
un module d'information de profondeur (211) configuré pour extraire des informations de profondeur à partir des données d'image ; et
un module de pose de pilote (213) configuré pour identifier une pluralité de points clés bidimensionnels correspondant aux caractéristiques des un ou plusieurs pilotes (P1, P2) dans les données d'image,
dans lequel le module de posture de pilote (214) est configuré pour combiner les informations de profondeur et les points clés bidimensionnels afin de déterminer la posture tridimensionnelle des un ou plusieurs pilotes dans le cockpit.

6. Procédé, comprenant :
la collecte, à l'aide d'un ou de plusieurs dispositifs d'imagerie (202), de données d'image d'une zone de cockpit d'un aéronef ;
la détermination, à l'aide d'un module de détection de pilote (212), d'une présence d'un ou de plusieurs pilotes dans la zone de cockpit sur la base des données d'image ;
la détermination, à l'aide d'un module de posture de pilote (214), d'une posture tridimensionnelle des un ou plusieurs pilotes (P1, P2) sur la base des données d'image ;
la détermination, à l'aide du module de posture de pilote (214), qu'un pilote est en train de tomber ou est tombé ;
la détermination, à l'aide d'un module d'image de détection d'objets (220), d'une présence et d'une position tridimensionnelle d'un ou de plusieurs objets dans la zone de cockpit sur la base des données d'image ;
la détermination, à l'aide d'un module pilote-objet (222), qu'un événement d'interaction pilote-objet s'est produit à la suite de la chute, sur la base de la posture tridimensionnelle des un ou plusieurs pilotes (P1, P2) et sur les positions tridimensionnelles des un ou plusieurs objets.

7. Procédé selon la revendication 6, comprenant :
le fait de vérifier, à l'aide d'un système d'alerte (120, 215), qu'une action a été enregistrée dans le journal de bord de système de vol de l'aéronef à la suite de l'événement d'interaction pilote-objet déterminé ; et
le fait d'alerter, à l'aide du système d'alerte (120, 215), un système de contrôle de l'aéronef que l'entrée d'action était un résultat de l'événement d'interaction pilote-objet déterminé.

8. Procédé selon les revendications 6 ou 7, comprenant :
l'alerte, à l'aide d'un système d'alerte (120, 215), à l'équipage d'aéronef et/ou au contrôle au sol et/ou au contrôle aérien qu'un ou plusieurs pilotes sont tombés.

9. Procédé selon l'une quelconque des revendications 6 à 8, comprenant :
l'extraction, à l'aide d'un module d'information de profondeur (211) du module de posture de pilote (214), d'informations de profondeur à partir des données d'image ;
l'identification, à l'aide d'un module de pose de pilote (213) du module de posture de pilote (214), d'une pluralité de points clés bidimensionnels correspondant aux caractéristiques des un ou plusieurs pilotes (P1, P2) dans les données d'image ; et
la combinaison, à l'aide du module de posture de pilote (214), des informations de profondeur et des points clés bidimensionnels afin de déterminer la posture tridimensionnelle des un ou plusieurs pilotes dans le cockpit.
